(19) Europäisches Patentamt
European Patent Office
Office européen des brevets

(11) Veröffentlichungsnummer: **0 443 406 A2**

(12) **EUROPÄISCHE PATENTANMELDUNG**

(21) Anmeldenummer: **91101910.7**

(22) Anmeldetag: **12.02.91**

(51) Int. Cl.[5]: **C12P 41/00**, C12P 17/04, C07C 229/12

(30) Priorität: **17.02.90 DE 4005150**

(43) Veröffentlichungstag der Anmeldung: **28.08.91 Patentblatt 91/35**

(84) Benannte Vertragsstaaten: **BE CH DE FR GB IT LI NL**

(71) Anmelder: **HOECHST AKTIENGESELLSCHAFT**
**Postfach 80 03 20**
**W-6230 Frankfurt am Main 80(DE)**

(72) Erfinder: **Fülling, Gerd, Dr.**
**Drosselweg 3**
**W-6230 Frankfurt am Main(DE)**
Erfinder: **Schudok, Manfred, Dr.**
**Birminghamstrasse 89**
**W-6230 Frankfurt am Main(DE)**

(54) **Verfahren zur enzymatischen Racematspaltung von Pantolacton.**

(57) (R)-(-)-Pantolacton kann durch enzymatische Racematspaltung von racemischem Pantolacton in Gegenwart eines Vinylesters und Lipase aus Pseudomonas sowie aus Schweinepankreas in hohen optischen Reinheiten hergestellt werden.

EP 0 443 406 A2

## VERFAHREN ZUR ENZYMATISCHEN RACEMATSPALTUNG VON PANTOLACTON

(R)-(-)-Pantolacton ((R)-(-)-$\beta,\beta$-Dimethyl-$\alpha$-hydroxy-$\tau$-butyrolacton) ist ein Zwischenprodukt in der Synthese von (R)-(+)-Pantothensäure, die zur Vitamin-B-Gruppe gehört und Bestandteil von Coenzym A ist.

Die Herstellung von (R)-(-)-Pantolacton kann auf verschiedene Arten durch Spaltung von racemischem Pantolacton erfolgen. Viele bekannte Verfahren beruhen auf einer Racematspaltung von racemischem Pantolacton mit optisch aktiven Hilfsstoffen (DE 2 404 305, U.S. 2,319,545, DE 1 568 755). Ferner ist die enzymatische Racematspaltung mit ausgewählten Enzymen durch eine stereoselektive Spaltung von racemischem O-Acetylpantolacton (Glänzer et al., Enzyme Microb. Technol., 10, 689 - 690, 1988) bekannt. Glänzer et al. stellten aber auch fest, daß eine Lipase aus Pseudomonas spec. (Amano Pharmaceuticals, Nagoya, Japan) sowie aus Schweinepanpkreas (Sigma Chem. Co. Typ II) nicht in der Lage ist, die Hydrolyse von racemischem O-Acetylpantolacton zu Essigsäure und Pantolacton zu katalysieren.

Überraschend wurde nun gefunden, daß sich die Umkehrreaktion, d.h. die enantioselektive Veresterung (O-Acylierung) von racemischem Pantolacton durch Lipasen aus Pseudomonas sowie aus Schweinepankreas (Serva, Heidelberg, BRD) katalysieren läßt, indem Vinylester oder Methylvinylester mit racemischem Pantolacton enzymkatalysiert umgesetzt werden. Dabei wird bevorzugt ein Enantiomer in den korrespondierenden Ester übergeführt

Die Erfindung betrifft somit ein Verfahren zur enzymatischen Racematspaltung von racemischem Pantolacton, das dadurch gekennzeichnet ist, daß der Vinylester der Formel I,

(I)

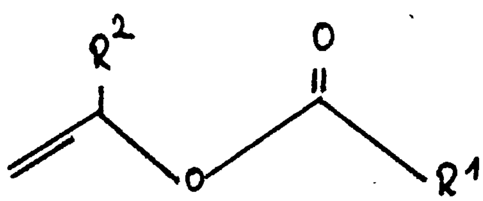

in der

$R^1$ für eine geradkettige oder verzweigte Alkylgruppe mit 1 bis 18 Kohlenstoffatomen, eine geradkettige oder verzweigte Halogenalkylgruppe mit 1 bis 18 Kohlenstoffatomen steht, wobei Halogen Chlor, Brom, Jod oder Fluor bedeutet oder eine Phenylgruppe, wobei die Phenylgruppe durch Chlor, Brom, Jod, Fluor, Cyanid, Nitro, Carboxy oder eine Alkoxygruppe mit 1 bis 4 Kohlenstoffatomen substituiert sein kann, und

$R^2$ für Wasserstoff oder eine Methylgruppe steht,

in Gegenwart von Lipase aus Pseudomonas oder Schweinepankreas mit racemischem Pantolacton der Formel II,

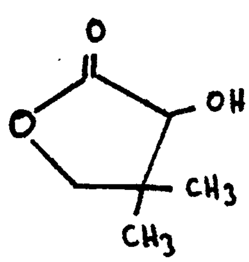

(II)

umgesetzt wird und anschließend das verbliebene optisch aktive (R)-(-)-Pantolacton von dem gebildeten Pantolactonester getrennt wird.

Die Erfindung wird im folgenden detailliert beschrieben, insbesondere in ihren bevorzugten Ausführungsformen.

Die erfindungsgemäße Racematspaltung von racemischem Pantolacton erfolgt in Gegenwart des Vinylesters der Formel I und Lipase aus Pseudomonas bzw. aus Schweinepankreas. Dazu kann man beispielsweise den Vinylester der Formel I, bevorzugt Vinylacetat oder Chloressigsäurevinylester, vorlegen

und sowohl die Lipase als auch racemisches Pantolacton dazugeben. Die Lipase kann auch in immobilisierter Form eingesetzt werden, wobei dafür alle gängigen Immobilisierungsmethoden und Träger in Frage kommen. Immobilisiertes und freies Enzym können auch im Säulenverfahren eingesetzt werden. Die verwendete Enzymmenge wird in Abhängigkeit von der Größe des Ansatzes, von der anzustrebenden Reaktionszeit und von der Art des Enzyms (frei oder fixiert) frei gewählt. Das Verhältnis von racemischem Pantolacton zu Lipase kann in einem weiten Bereich schwanken. Beispielsweise kann für eine Pseudomonas Lipase mit 30 000 U/g; (U = $\mu$mol Sesamöl/ min) das Verhältnis von racemischem Pantolacton zu Lipase (Volumen zu Gewicht) im Bereich von 50:1 bis 1:100, vorzugsweise im Bereich von 10:1 bis 2:1, schwanken. Entsprechende Bereiche für das Verhältnis von Pantolacton zu anderen Lipasen können nach der spezifischen Aktivität der Lipase berechnet werden. Einem Fachmann fällt es nicht schwer in einfachen Vorversuchen die optimale Menge zu bestimmen.

Das racemische Pantolacton wird in Gewichtsmengen von 0,01 bis 2 zu 1, bevorzugt von 0,1 bis 1,5 zu 1 - bezogen auf das Volumen des eingesetzten Vinylesters der Formel I - verwendet. In manchen Fällen ist es auch möglich, den Vinylester nur in stöchiometrischen Mengen - bezogen auf ein Enantiomer - vorzulegen. Zur Reaktionslösung können auch wahlweise noch andere unpolare Lösungsmittel zugesetzt werden, beispielsweise tertiär-Butylmethylether, Hexan, Isooctan, Diethylether oder Dipropylether.

Die Reaktionstemperatur liegt in einem Bereich von 10 °C bis 80 °C, bevorzugt zwischen 20 °C und 50 °C. Es ist zweckmäßig, die Lösung während der Reaktion zu rühren. Die Reaktionszeiten schwanken in Abhängigkeit von der verwendeten Enzymmenge zwischen wenigen Stunden und bis zu 4 Wochen. Sie liegen häufig zwischen 3 Stunden und 14 Tagen.

Der Vinylester der Formel I ist, sofern er nicht käuflich ist, auf einfache Weise herstellbar. Die Vinylester der Formel I können beispielsweise durch metallkatalysierte Umesterung von Vinylacetat mit den entsprechenden Carbonsäuren gewonnen werden (Geckeler et al., Chemische Berichte, 107, 1271-1274, 1974). Racemisches Pantolacton ist käuflich zu erwerben (Sigma Chemie GmbH, FRG). Lipase aus Pseudomonas kann beispielsweise als Lipase P, Lipase FP oder Lipase PS (Amano Pharmaceuticals, Nagoya, Japan) käuflich erworben werden. Schweinepankreas-Lipase ist ebenfalls käuflich erhältlich (Sigma Chem. Co. Typ II).

Beim erfindungsgemäßen Verfahren entstehen (R)-(-)-Pantolacton, der je nach dem verwendeten Vinylester der Formel I entsprechende Pantolactonester, beispielsweise (S)-O-Acyl-pantolacton oder (S)-O-Halogenacylpantolacton und Acetaldehyd bzw. Aceton als Reaktionsprodukte. Diese Produkte lassen sich in bekannter Art und Weise trennen. Im Idealfall läßt sich (R)-(-)-Pantolacton durch Destillation gewinnen. Ist die destillative Trennung nicht möglich, so erfolgt die Trennung chromatographisch, extraktiv oder durch Kristallisation. Es besteht auch die Möglichkeit, die Siedepunktdifferenz zwischen dem enzymatisch gebildeten (S)-Pantolactonester und dem nicht umgesetzten (R)-(-)-Pantolacton dadurch zu erhöhen, daß anstelle von Vinylester der entsprechende Halogenalkylvinylester eingesetzt wird, beispielsweise Chloracetylvinylester.

Soll auch das andere Enantiomer von Pantolacton rein dargestellt werden, so isoliert man zunächst den Pantolactonester und spaltet diesen auf bekannte Weise in Säure und Alkohol.

Die für die Reaktion verwendeten Lipasen lassen sich leicht durch Filtration, Zentrifugation oder verwandte Verfahren wieder zurückgewinnen.

Die optischen Ausbeuten lassen sich auf bekannte Weise durch Kristallisation (DE 2 404 305) oder durch Variation der Reaktionszeit bzw. des Umsatzes verbessern (Sih et al., J. Am. Chem. Soc., 104, 7294-7299, 1982).

Die nachfolgend aufgeführten Beispiele dienen der weiteren Erläuterung der Erfindung.

**Beispiel:**

1) 20 g (R,S)-Pantolacton werden in 200 ml Vinylacetat gelöst und nach Zugabe von 10.g Lipase P aus Pseudomonas für 10 Tage bei Raumtemperatur gerührt. Die Reaktion wird per Dünnschichtchromatographie verfolgt. Nach Beendigung der Reaktion (50 % Umsatz) filtriert man das Enzym ab, das in der anfallenden Form wiederholt eingesetzt werden kann. Die verbliebene Lösung wird im Vakuum bis zur Trockene eingeengt.

Die Trennung von (R)-(-)-Pantolacton und (S)-(+)-O-Acetyl-pantolacton erfolgt durch Säulenchromatographie an Kieselgel (Laufmittel Hexan und Essigester im Volumenverhältnis von 5:1). Die (R)-(-)-Pantolacton und (S)-(+)-O-Acetylpantolacton enthaltenden Fraktionen werden jeweils vereinigt und die Lösungen werden im Vakuum bis zur Trockene eingeengt.

Ausbeute:

11,4 g (S)-(+)-O-Acetylpantolacton

$[\alpha]_D^{20}$ = + 12,4 (c=1, $CHCl_3$)
ee = 85 % (bestimmt durch $^1$H-NMR mit Zugabe von Shiftreagenz Tris-[3-( heptafluoropropylhydroxymethylen)-( + )-camphorato]-europium (III)

8,0 g (R)-(-)-Pantolacton
$[\alpha]_D^{20}$ = - 42,5 (c=1 in $H_2O$)
ee = 85 % (Bestimmung wie oben)

2) Die Reaktion wird analog Beispiel 1 jedoch mit unterschiedlichen Ausgangsmengen durchgeführt. Es werden 0,2 g (R,S)-Pantolacton in 5 ml Vinylacetat gelöst und nach Zugabe von 0,2 g Lipase P aus Pseudomonas für 10 Tage bei Raumtemperatur gerührt.
Der Fortgang der Reaktion wird über den per Gaschromatographie bestimmten Umsatz kontrolliert.
Messung des Umsatzes über GC:
Eine Glassäule (2m) wird mit calciniertem Kieselgur (z.B. Reoplex auf ®Chromosorb, WZ Johns-Manville) gefüllt. Die Säule wird auf 100 °C aufgeheizt und nach Aufgabe der Probe für zunächst 0,5 min bei dieser Temperatur gehalten, bevor mit einer Geschwindigkeit von 10 °C/min bis zur Endtemperatur von 135 °C aufgeheizt wird. Die Detektion erfolgt mit Hilfe eines Flammenionisationsdetektors (FID).
Die Retentionszeiten betragen für

- Pantolacton: 2,7 min
- Pantolacton-acetat: 3,1 min

GC-Umsatz (% Fläche) nach:

- 48 Std: 29,80
- 72 Std: 37,70
- 96 Std: 42,44
- 168 Std: 49,90
- 216 Std: 53,53

3) Entsprechend dem in Beispiel 1 angegebenen Versuchsablauf kann die Reaktion auch mit der kommerziell erhältlichen Lipase aus Schweinepankreas durchgeführt werden.
Die Reaktion wird analog Beispiel 1, jedoch mit unterschiedlichen Ausgangsmengen durchgeführt. Es werden 0,2 g (R,S)-Pantolacton in 5 ml Vinylacetat gelöst und nach Zugabe von 0,2 g Lipase aus Schweinepankreas für 9 Tage bei Raumtemperatur gerührt.
Die Bestimmungsmethoden für Umsatz und Ausbeute stimmten ebenfalls mit denen von Beispiel 1 und 2 überein.
Die Reaktion wurde mittels Gaschromatographie verfolgt.
Die Messung des Umsatzes erfolgt über GC.
GC-Umsatz (% Fläche) nach:

- 48 Std: 3,52
- 72 Std: 5,34
- 96 Std: 7,59
- 168 Std: 7,82
- 216 Std: 11,51

Ausbeute:
30 mg (S)-( + )-O-Acetylpantolacton

165 mg (R)-(-)-Pantolacton

Beispiele 4 und 5

Die Reaktion wird analog Beispiel 1 bei verschiedenen Temperaturen durchgeführt. Die Ergebnisse sind in Tabelle 1 zusammengefaßt.

Beispiele 6 - 9

Die Reaktion wird analog Beispiel 1 unter Zusatz diverser Lösungsmittel durchgeführt. Die Ergebnisse sind in Tabelle 2 zusammengestellt.

Tabelle 1

Einfluß der Temperatur auf die Reaktionsgeschwindigkeit der enantiodifferenzierenden, lipasekatalysierten Veresterung (O-Acylierung) von (±)-Pantolacton

| Bsp. | Einwaage: Pantolacton | Vinylacetat | Lipase P | Reaktionszeit | Temp. | GC-Umsatz | Ausbeute<br>a) (S)-(+)-O-Acetyl-pantolacton<br>b) (R)-(-)-Pantolacton |
|---|---|---|---|---|---|---|---|
| 4 | 0,4 g | 20 ml | 2 g | 24 h | 22°C | 25 % | a) 115 mg<br>b) 275 mg |
| 5 | 0,4 g | 20 ml | 2 g | 24 h | 50°C | 48 % | a) 228 mg<br>b) 175 mg |

Tabelle 2

Einfluß von Lösungsmittelzusätzen auf die Reaktionsgeschwindigkeit der lipasekatalysierten Veresterung (O-Acylierung) von (±)-Pantolacton

| Bsp. | Einwaage: Pantolacton | Lösemittel (jeweils 15 ml) | Vinylacetat | Lipase P | Reaktionszeit | GC-Umsatz | Ausbeute a)(S)-(+)-O-Acetyl-pantolacton b)(R)-(-)-Pantolacton |
|---|---|---|---|---|---|---|---|
| 6 | 0,25 g | - | 15 ml | 1 g | 24 h | 36,2 % | a) 104 mg<br>b) 141 mg |
| 7 | 0,25 g | Hexan | 0,25 ml | 1 g | 24 h | 32,9 % | a) 95 mg<br>b) 145 mg |
| 8 | 0,25 g | Ether | 0,25 ml | 1 g | 24 h | 30,6 % | a) 92 mg<br>b) 165 mg |
| 9 | 0,25 g | t-Butylmethyl-ether | 0,25 ml | 1 g | 24 h | 23,1 % | a) 51 mg<br>b) 171 mg |

**Patentansprüche**

1. Verfahren zur enzymatischen Racematspaltung von racemischem Pantolacton, dadurch gekennzeichnet, daß der Vinylester der Formel I,

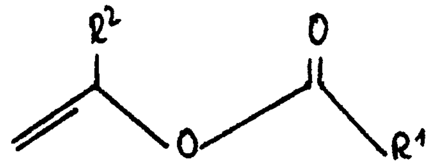

(I)

in der

$R^1$ für eine geradkettige oder verzweigte Alkylgruppe mit 1 bis 18 Kohlenstoffatomen, eine geradkettige oder verzweigte Halogenalkylgruppe mit 1 bis 18 Kohlenstoffatomen steht, wobei Halogen Chlor, Brom, Jod oder Fluor bedeutet oder eine Phenylgruppe, wobei die Phenylgruppe durch Chlor, Brom, Jod, Fluor, Cyanid, Nitro, Carboxy oder eine Alkoxygruppe mit 1 bis 4 Kohlenstoffatomen substituiert sein kann, und

$R^2$ für Wasserstoff oder eine Methylgruppe steht,

in Gegenwart von Lipase aus Pseudomonas oder Schweinepankreas mit racemischem Pantolacton der Formel II,

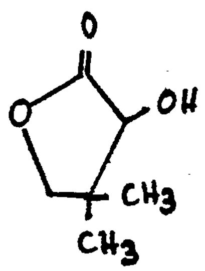

(II)

umgesetzt wird und anschließend das verbliebene optisch aktive (R)-(-)-Pantolacton von dem gebildeten Pantolactonester getrennt wird.

2. Verfahren nach Anspruch 1, dadurch gekennzeichnet, daß als Vinylester Vinylacetat oder Chloressigsäurevinylester verwendet wird.

3. Verfahren nach Anspruch 1 oder 2, dadurch gekennzeichnet, daß das Gewichtsverhältnis von racemischem Pantolacton zu Vinylester zwischen 0,01 und 2 zu 1 liegt.

4. Verfahren nach Anspruch 3, dadurch gekennzeichnet, daß das Gewichtsverhältnis von racemischem Pantolacton zu Vinylester zwischen 0,1 und 1,5 zu 1 liegt.

5. Verfahren nach einem oder mehreren der Ansprüche 1 bis 4, dadurch gekennzeichnet, daß das Verfahren bei einer Temperatur zwischen 10 °C und 80 °C durchgeführt wird.

6. Verfahren nach Anspruch 5, dadurch gekennzeichnet, daß das Verfahren bei einer Temperatur zwischen 20 °C und 50 °C durchgeführt wird.

7. Verwendung des gemäß einem oder mehreren der Ansprüche 1 bis 6 erhältlichen (R)-(-)-Pantolacton zur Herstellung von (R)-(+)-Pantothensäure.